Europäisches Patentamt

European Patent Office

Office européen des brevets

(11) Publication number: **0 049 468**
**B1**

(12) **EUROPEAN PATENT SPECIFICATION**

(45) Date of publication of patent specification: **16.05.84**

(21) Application number: **81107769.2**

(22) Date of filing: **30.09.81**

(51) Int. Cl.³: **A 61 K 31/57,** A 61 K 31/71, A 61 K 9/00 // (A61K31/71, 31/57, 31/415)

(54) Antifungal compositions comprising an antifungal agent and a corticosteroid.

(30) Priority: **06.10.80 US 194524**

(43) Date of publication of application:
**14.04.82 Bulletin 82/15**

(45) Publication of the grant of the patent:
**16.05.84 Bulletin 84/20**

(84) Designated Contracting States:
**AT BE CH DE FR GB IT LI NL SE**

(56) References cited:
**EP-A-0 007 595**
**FR-A-2 368 960**

**ARZNEIMITTEL-FORSCHUNG, vol. 28 (I), no. 2, February 1978 AULENDORF (DE) CH. POITSCHECK et al.: "Bestimmung der antimikrobiellen Wirksamkeit einer Kombination aus Azidamfenicol, Clotrimazol und Dexamethason in vitro" pages 232-234**
**UNLISTED DRUGS, vol. 27, no. 1, January 1975, CHATHAM/NEW YORK (US) page 5**
**UNLISTED DRUGS, vol. 26, no. 9, September 1974, CHATHAM/NEW YORK (US) page 142**
**DICTIONNAIRE VIDAL, 1975, O.V.P. PARIS (FR) 51st. Edition page 536**
**MARTIN MEGWER: "ORGANISCH-CHEMISCHE ARZNEIMITTEL UND IHRE SYNONYMA" vol. 2, 1978 Akademie Verlag BERLIN (DE) page 1063**

(73) Proprietor: **SCHERING CORPORATION**
**2000 Galloping Hill Road**
**Kenilworth, New Jersey 07033 (US)**

(72) Inventor: **Hammell, Susan B.**
**41 Eaton Court**
**Berkeley Heights New Jersey 07922 (US)**

(74) Representative: **Antony, Fritz, Dr. et al**
**P.O. Box 601 Winkelriedstrasse 35**
**CH-6002 Lucerne (CH)**

Courier Press, Leamington Spa, England.

## Description

The present invention relates to antifungal compositions comprising a synthetic antifungal agent and a corticosteroid.

According to the present invention there are provided compositions comprising 0.001 to 1.0 percent by weight betamethasone dipropionate and 0.01 to 10 percent by weight clotrimazole wherein the ratio of clotrimazole to betamethasone dipropionate is in the range of (10—30):1, in a pharmaceutically acceptable carrier. The compositions of the present invention preferably comprise 0.01 to 0.33 percent, more preferably 0.01 to 0.1 percent by weight betamethasone dipropionate and 0.1 to 2 percent by weight clotrimazole. A particularly preferred composition comprises substantially 0.06 percent by weight betamethasone dipropionate and substantially 1 percent by weight clotrimazole.

The compositions of the present invention are surprisingly more effective against fungi than compositions comprising only clotrimazole and may be used in the topical treatment of a variety of fungal infections, in particular in the treatment of the following dermal and/or vaginal infections: tinea pedis, tinea cruris, and tinea corporis due to, for example, *Trichophyton rubrum, Trichophyton menta-grophytes, Epidermophyton floccosum,* and *Microsporum canis;* candidiasis due to *Candida albicans;* and tinea versicolor due to *Malassezia furfur.* The compositions of the present invention are particularly useful when such injections are accompanied by moderate to severe inflammation.

Depending on the severity of the infection, the present compositions may be administered one or more times per day for a period ranging from a few days to several weeks until sufficient improvement is obtained in the judgment of the attending clinician. The compositions may be administered topically in the form of creams, ointments, lotions, solutions, aerosol sprays, and the like. Although an ointment, owing to the occlusive effect of the anhydrous petrolatum, is likely to be the most effective vehicle, a cream or lotion is likely to more esthetically acceptable to the patient. For treatment of vaginal infections, the use of foaming tablets, foam sprays or suppositories may also be advantageous. Coated condoms may be useful for prophylaxis, in particular in preventing the transmission of fungal infections.

If desired, the compositions according to the present invention can contain further active ingredients compatible with clotrimazole and betamethasone diproprionate. These further active ingredients may for example extend the antibiotic spectrum of the clotrimazole or be synergistic with the clotrimazole or with the betamethasone dipropionate. In particular, further topically active antibiotics and/or anti-inflammatory agents may be present. For example, such antibiotics may be pseudopolysaccharides such as neomycin, especially pseudotrisaccharides such as gentamicin, if desired used as a pharmaceutically acceptable acid addition salt such as the sulfate.

The following example gives illustrative formulations, at five concentrations, of the compositions of the present invention. All weights are given in milligrams. Cream, ointment and lotion formulations having the same identifying letter have the same concentration of active ingredients. The following ingredients are combined and packaged by standard techniques well-known to those skilled in the art, to give the cream, ointment or lotion as required. A dash in column B, C, D or E indicates that the weight of a particular ingredient is the same as that in column A.

### Example

| Cream | A | B | C | D | E |
|---|---|---|---|---|---|
| Betamethasone dipropionate | 0.0100 | 10.0 | 0.100 | 1.00 | 0.643 |
| Clotrimazole | 0.100 | 100 | 1.00 | 20.0 | 10.0 |
| Mineral oil | 60.0 | — | — | — | — |
| Petrolatum | 150 | — | — | — | — |
| Cetostearyl alcohol | 72.0 | — | — | — | — |
| Polyethylene glycol 1000 monocetyl ether | 22.5 | — | — | — | — |
| Benzyl alcohol | 10.0 | — | — | — | — |
| Sodium phosphate monobasic monohydrate | 2.65 | — | — | — | — |
| Phosphoric acid | 0.0200 | — | — | — | — |

2

| Cream | A | B | C | D | E |
|---|---|---|---|---|---|
| Propylene glycol | 100 | — | — | — | — |
| Purified water q.s. 1 gram | | | | | |

| Ointment | A | B | C | D | E |
|---|---|---|---|---|---|
| Betamethasone dipropionate | 0.0100 | 10.0 | 0.100 | 1.00 | 0.643 |
| Clotrimazole | 0.100 | 100 | 1.00 | 20.0 | 10.0 |
| Mineral oil | 50.0 | — | — | — | — |
| Petrolatum q.s. 1 gram | | | | | |

| Lotion | A | B | C | D | E |
|---|---|---|---|---|---|
| Betamethasone dipropionate | 0.0100 | 10.0 | 0.100 | 1.00 | 0.643 |
| Clotrimazole | 0.100 | 100 | 1.00 | 20.0 | 10.0 |
| Mineral oil | 10.0 | — | — | — | — |
| Cetostearyl alcohol | 3.00 | — | — | — | — |
| Polyethylene glycol 1000 monocetyl ether | 10.0 | — | — | — | — |
| Benzyl alcohol | 10.0 | — | — | — | — |
| Sodium phosphate monobasic monohydrate | 2.65 | — | — | — | — |
| Phosphoric acid | 0.0200 | — | — | — | — |
| Propylene glycol | 100 | — | — | — | — |
| Purified water q.s. 1 gram | | | | | |

| Ointment containing gentamicin sulfate | |
|---|---|
| Betamethasone diproprionate | 0.643 |
| Clotrimazole | 10.0 |
| Gentamicin sulfate equivalent to gentamicin base | 1.0 |
| Mineral oil U.S.P. | 50.0 |
| White pertolatum U.S.P. q.s. 1 gram | |

The following Tables illustrate the advantageous activity of compositions according to the present invention:

3

# 0 049 468

TABLE 1

In Vivo Activities of Various Clotrimazole Formulations Applied
Topically to Guinea Pigs Infected with T. mentagrophytes

Treatment Twice Daily for 10 Days

| Group and Treatment | Cultures Negative | Lesion Scores | | | Average Day to Become Negative | |
| --- | --- | --- | --- | --- | --- | --- |
| | | Total | Significant From Group | P= | Number of Animals | Day |
| 1. 'Diprosone' Cream plus Clotrimazole | 100% | 19.0 | 2 and 3 | .01 | 5 | 2 |
| 2. 'Lotrimin' Cream* | 100% | 26.9 | 1 and 3 | .01 | 5 | 2 |
| 3. Untreated Controls | 0% | 30.6 | | | 5 | >16 |

* 'Lotrimin' cream: active ingredient is clotrimazole, 1%.

Dose: Diprosane 0.064%
      Clotrimazole 1%

Notes: (i) "Lotrimin" and "Diprosone" are Trade Marks
      (ii) The vanishing cream base of "Lotrimin" cream contains sorbitan monostearate, polysorbate 60, cetyl esters wax, cetostearyl alcohol, 2-octyldodecanol, purified water and benzyl alcohol.

TABLE 2

In Vivo Activities of Various Clotrimazole Formulations Applied
Topically to Guinea Pigs Infected with T. mentagrophytes

Treatment Twice Daily for 10 Days

| Group and Treatment | Cultures Negative | Lesion Scores | | | Average Day to Become Negative | |
| --- | --- | --- | --- | --- | --- | --- |
| | | Total | Significant From Group | P= | Number of Animals | Day |
| 1. Diprosone Ointment plus Clotrimazole | 100% | 5.0 | 2 and 3 | .01 | 5 | 2 |
| 2. Commercial 'Lotrimin' * | 100% | 18.1 | 3 | .10 | 5 | 2 |
| 3. Untreated Controls | 0% | 22.5 | | | 10 | >16 |

* Active ingredient: clotrimazole, 1%.

Dose: Diprosone 0.064%
      Clotrimazole 1%

**Claims for the Contracting States: BE CH DE FR GB IT LI NL SE**

1. A pharmaceutical composition comprising 0.001 to 1.0 percent by weight betamethasone dipropionate and 0.01 to 10 percent by weight clotrimazole, wherein the ratio of clotrimazole to betamethasone dipropionate is in the range of (10—30):1, in a pharmaceutically acceptable carrier.

2. A composition as claimed in claim 1 wherein the concentration of betamethasone dipropionate is 0.01 to 0.33 percent by weight.

3. A composition as claimed in claim 2 where the concentration of betamethasone dipropionate is 0.01 to 0.1 percent by weight and the concentration of clotrimazole is 0.1 to 2 percent by weight.

4. A composition as claimed in claim 1 wherein the concentration of betamethasone dipro-

4

pionate is substantially 0.06 percent by weight and the concentration of clotrimazole is substantially 1 percent by weight.

5. A composition as claimed in any of claims 1 to 4 in the form of a cream.

6. A composition as claimed in any of claims 1 to 4 in the form of an ointment.

7. A composition as claimed in any of claims 1 to 4 in the form of a lotion.

8. A composition as claimed in any of claims 1 to 4 in the form of a solution, aerosol spray, foaming tablet or suppository.

9. A composition as claimed in any of claims 1 to 8 containing an additional topically active antibiotic and/or anti-inflammatory agent.

10. A composition as claimed in claim 9 containing gentamicin or a pharmaceutically acceptable acid addition salt thereof.

**Claims for the Contracting State: AT**

1. A process for the preparation of a pharmaceutical composition comprising betamethasone dipropionate and clotrimazole in a pharmaceutical carrier, characterised in that 0.001 to 1.0 percent by weight betamethasone dipropionate and 0.01 to 10 percent by weight clotrimazole, the ratio of clotrimazole to betamethasone dipropionate being in the range of (10—30):1, is admixed with a pharmaceutically acceptable carrier.

2. A process as claimed in claim 1, characterised in that 0.01 to 0.33, preferably 0.01 to 0.1, percent by weight betamethasone dipropionate and 0.1 to 2 percent by weight clotrimazole is admixed with a pharmaceutically acceptable carrier.

3. A process as claimed in claim 1, characterised in that substantially 0.06 percent by weight betamethasone dipropionate and substantially 1 percent by weight clotrimazole is admixed with a pharmaceutically acceptable carrier.

4. A process as claimed in any of claims 1 to 3, characterised in that the composition is produced in the form of a cream.

5. A process as claimed in any of claims 1 to 3, characterised in that the composition is produced in the form of an ointment.

6. A process as claimed in any of claims 1 to 3, characterised in that the composition is produced in the form of a lotion.

7. A process as claimed in any of claims 1 to 3, characterised in that the composition is produced in the form of a solution, aerosol spray, foaming tablet or suppository.

**Revendications pour les Etats contractants: BE CH DE FR GB IT LI NL SE**

1. Composition pharmaceutique comprenant 0,001 à 1,0 pour cent en poids de dipropionate de bétaméthasone et 0,01 à 10 pour cent en poids de clotrimazole, où le rapport de clotrimazole au dipropionate de bétaméthasone est compris entre (10—30):1, dans un véhicule acceptable en pharmacie.

2. Composition selon la revendication 1 où la concentration du dipropionate de bétaméthasone est de 0,01 à 0,33 pour cent en poids.

3. Composition selon la revendication 2 où la concentration en dipropionate de bétaméthasone est de 0,01 à 0,1 pour cent en poids et la concentration en clotrimazole est de 0,1 à 2 pour cent en poids.

4. Composition selon la revendication 1 où la concentration en dipropionate de bétaméthasone est sensiblement de 0,06 pour cent en poids et la concentration en clotrimazole est sensiblement de 1 pour cent en poids.

5. Composition selon l'une quelconque des revendications 1 à 4 sous la form d'une crème.

6. Composition selon l'une quelconque des revendications 1 à 4 sous la forme d'un onguent.

7. Compositon selon l'une quelconque des revendications 1 à 4 sous la forme d'une lotion.

8. Composition selon l'une quelconque des revendications 1 à 4 sous la forme d'une solution, d'une pulvérisation en aérosol, d'un comprimé moussant ou d'un suppositoire.

9. Compositon selon l'une quelconque des revendications 1 à 8 contenant en supplément un antibiotique topiquement actif et/ou agent anti-inflammatoire.

10. Composition selon la revendication 9 contenant de la gentamicine ou son sel d'addition d'acide acceptable en pharmacie.

**Revendications pour l'Etat contractant: AT**

1. Procédé de préparation d'une composition pharmaceutique comprenant du dipriopionate de bétaméthasone et du clotrimazole dans un véhicule pharmaceutique, caractérisé en ce qu'on mélange 0,001 à 1,0 pour cent de dipriopionate de bétaméthasone et 0,01 à 10 pour cent en poids de clotrimazole, le rapport du clotrimazole au dipriopionate de bétaméthasone étant de l'ordre de (10—30):1, à un véhicule acceptable en pharmacie.

2. Procédé selon la revendication 1 caractérisé en ce qu'on mélange 0,01 à 0,33, de préférence

5

0,01 à 0,1 pour cent en poids de dipropionate de bétaméthasone et 0,1 à 2 pour cent en poids de clotrimazole à un véhicule acceptable en pharmacie.

3. Procédé selon la revendication 1, caractérisé en ce qu'on mélange sensiblement 0,06 pour cent en poids de dipropionate de bétaméthasone et sensiblement 1 pour cent en poids de clotrimazole à un véhicule acceptable en pharmacie.

4. Procédé selon l'une quelconque des revendications 1 à 3, caractérisé en ce que la composition est produite sous forme d'une crème.

5. Procédé selon l'une quelconque des revendication 1 à 3, caractérisé en ce que la composition est produite sous la forme d'un onguent.

6. Procédé selon l'une quelconque des revendications 1 à 3, caractérisé en ce que la composition est produite sous la forme d'une lotion.

7. Procédé selon l'une quelconque des revendication 1 à 3, caractérisé en ce que la composition est produite sous forme d'une solution, d'une pulvérisation en aérosol, d'un comprimé moussant ou d'un suppositoire.

**Patentansprüche für die Vertragsstaaten: BE CH DE FR GB IT LI NL SE**

1. Pharmazeutische Zusammensetzung, enthaltend 0,001 bis 1,0 Gew.-% Betamethason-dipropionat und 0,01 bis 10 Gew.-% Clotrimazol, wobei das Verhältnis des Clotrimazols zu dem Betamethason-dipropionat in dem bereich von (10—30):1 liegt, in einem pharmazeutisch unbedenklichen Träger.

2. Zusammensetzung nach Anspruch 1, dadurch gekennzeichnet, daß die Konzentration des Betamethason-dipropionats 0,01 bis 0,33 Gew.-% beträgt.

3. Zusammensetzung nach Anspruch 2, dadurch gekennzeichnet, daß die Konzentration des Betamethason-dipropionats 0,01 bis 0,1 Gew.-% und die Konzentration des Clotrimazoles 0,1 bis 2 Gew.-% beträgt.

4. Zusammensetzung nach Anspruch 1, dadurch gekennzeichnet, daß die Konzentration des Betamethason-dipropionats im wesentlichen 0,06 Gew.-% und die Konzentration des Clotrimazols im wesentlichen 1 Gew.-% beträgt.

5. Zusammensetzung nach einem der Ansprüche 1 bis 4 in Form einer Creme.

6. Zusammensetzung nach einem der Ansprüche 1 bis 4 in Form einer Salbe.

7. Zusammensetzung nach einem der Ansprüche 1 bis 4 in Form einer Lotion.

8. Zusammensetzung nach einem der Ansprüche 1 bis 4 in Form einer Lösung, eines Aerosol-Sprays, einer schäumenden Tablette oder eines Suppositoriums.

9. Zusammensetzung nach einem der Ansprüche 1 bis 8, dadurch gekennzeichnet, daß sie ein zusätzliches, topisch wirksames Antibiotikum und/oder antiinflammatorisches Mittel enthält.

10. Zusammensetzung nach Anspruch 9, dadurch gekennzeichnet, daß sie Gentamicin oder pharmazeutisch unbedenkliches Säureadditionssalz desselben enthält.

**Patentansprüche für den Vertragsstaat: AT**

1. Verfahren zur Herstellung einer pharmazeutischen Zusammensetzung, die Betamethason-dipropionat und Clotrimazol in einem pharmazeutischen Träger enthält, dadurch gekennzeichnet, daß 0,001 bis 1,0 Gew.-% Betamethason-dipropionat und 0,01 bis 10 Gew.-% Clotrimazole, wobei das Verhältnis des Clotrimazols zu dem Betamethason-dipropionat in dem Bereich von (10—30):1 liegt, mit einem pharmazeutisch unbedenklichen Träger vermischt werden.

2. Verfahren nach Anspruch 1, dadurch gekennzeichnet, daß 0,01 bis 0,33, vorzugsweise 0,01 bis 0,1 Gew.-% Betamethason-dipropionat und 0,1 bis 2 Gew.-% Clotrimazol mit einem pharmazeutisch unbedenklichen Träger vermischt werden.

3. Verfahren nach Anspruch 1, dadurch gekennzeichnet, daß im wesentlichen 0,06 Gew.-% Betamethason-dipropionat und im wesentlichen 1 Gew.-% Clotrimazol mit einem pharmazeutisch unbedenklichen Träger vermischt werden.

4. Verfahren nach Anspruch 1 bis 3, dadurch gekennzeichnet, daß die Zusammensetzung in Form einer Creme hergestellt wird.

5. Verfahren nach einem der Ansprüche 1 bis 3, dadurch gekennzeichnet, daß die Zusammensetzung in Form einer Salbe hergestellt wird.

6. Verfahren nach einem der Ansprüche 1 bis 3, dadurch gekennzeichnet, daß die Zusammensetzung in Form einer Lotion hergestellt wird.

7. Verfahren nach einem der Anspüche 1 bis 3, dadurch gekennzeichnet, daß die Zusammensetzung in Form einer Lösung, eines Aerosol-Sprays, einer schäumenden Tablette oder eines Suppositoriums hergestellt wird.